# EUROPEAN PATENT APPLICATION

(11) **EP 4 737 072 A1**
(43) Date of publication of application: **06.05.2026**
(21) Application number: 23945147.9
(22) Date of filing: 13.07.2023
(51) Int. Cl.: B25J 13/02, G05G 1/00, G05G 1/04, A61B 34/30

(54) **HAND CONTROLLER AND CONSOLE DEVICE**

(71) Applicant: Riverfield Inc., Tokyo 107-0052 (JP)
(72) Inventor: TANAKA Yasushi, Tokyo 107-0052 (JP)
(74) Representative: Adares PartGmbB
(86) International application number: PCT/JP2023/025844
(87) International publication number: WO 2025/013274

(57) **Abstract**

The purpose of one or more embodiments of the present disclosure is to prevent mutual contact of left and right articulated link mechanisms. The tab has a first side surface and a second side surface that are oriented oppositely from each other, and has an end surface that is provided from the first side surface to the second side surface. The handle is positioned facing the end surface of the tab, and is attached The operation part is positioned facing the first side surface of the tab, and is attached to the tab so as to come into contact with and separate from the end surface of the tab. The operation part is positioned facing the first side surface of the tab, and is linked to the tab so as to come into contact with and separate from the first side surface of the tab. sensor is provided to the second side surface of the tab, and detects a finger of a hand of an operator.

## Description

### TECHNICAL FIELD

This disclosure relates to a hand controller and a console device.

### BACKGROUND ART

Patent Document 1 discloses a system for operating a medical device using an input control console. A sensor detects whether an operator is in contact with the input control console. When the sensor detects operator contact with the input control console, a computer processor issues commands to the medical device based on the operator's manipulation of the input control console. When the sensor does not detect operator contact with the input control console, the computer processor blocks commands to the medical device.

### CITATION LIST

### Patent Literature

Patent Document 1: Japanese Translation of PCT International Application Publication No. JP-T-2020-529237

### SUMMARY OF INVENTION

### Technical Problem

A sensor may be provided on a controller to detect that the operator holds the controller of the console device by hand. If the size of the controller does not match the size of the hand, the hand will not contact the sensor, and the sensor will fail to detect the hand. No matter how much the operator manipulates the controller, commands to a robot, such as medical equipment, will be blocked, and the robot will not operate.

Therefore, it is an object of one or more embodiments of the present disclosure to ensure that the fingers of the operator's hand are detected by a sensor, regardless of whether the operator's hand is large or small.

### Solution to Problem

To solve the above problem, according to an aspect of the present disclosure, a hand controller comprises a tab, a handle, an operation element, and a finger sensor. The tab has a first side surface and a second side surface facing away from each other, and an end surface that extends from the first side surface to the second side surface. The handle is disposed facing the end surface of the tab and mounted to the tab to allow the handle to be moved in and out of contact with the end surface of the tab. The operation element is disposed facing the first side surface of the tab and coupled to the tab to allow the operation element to be moved in and out of contact with the first side surface of the tab. The finger sensor is disposed on the second side surface of the tab and detects a finger of a hand of an operator.

According to another aspect of the present disclosure, a console device comprises the hand controller, a foot switch, an articulated link mechanism, and a controller. The foot switch outputs a trigger when pressed by a foot of the operator. The articulated link mechanism has a pivotable proximal end and a distal end to which the hand controller is connected and translatably supports the hand controller. The controller switches between an operation mode in which the controller is capable of controlling a robot and a standby mode in which the controller is incapable of controlling the robot. When the finger sensor detects the finger of the hand and the controller receives the trigger from the foot switch, the controller switches the robot between the operation mode and the standby mode

### Advantageous Effects of Invention

The hand controller and the console device of one or more embodiments of the present disclosure contribute to the operator being able to grasp the handle with his/her palm and place his/her finger on the finger sensor, regardless of the size of the operator's hand. The fingers of the operator's hand are detected by the finger sensor, regardless of whether the operator's hand is large or small.

### BRIEF DESCRIPTION OF DRAWINGS

[FIG. 1] This is a block diagram illustrating a remote-controlled robot system.
[FIG. 2] This illustrates a console device.
[FIG. 3] This illustrates left and right consoles of the console device.
[FIG. 4] This illustrates the left and right consoles of the console device.

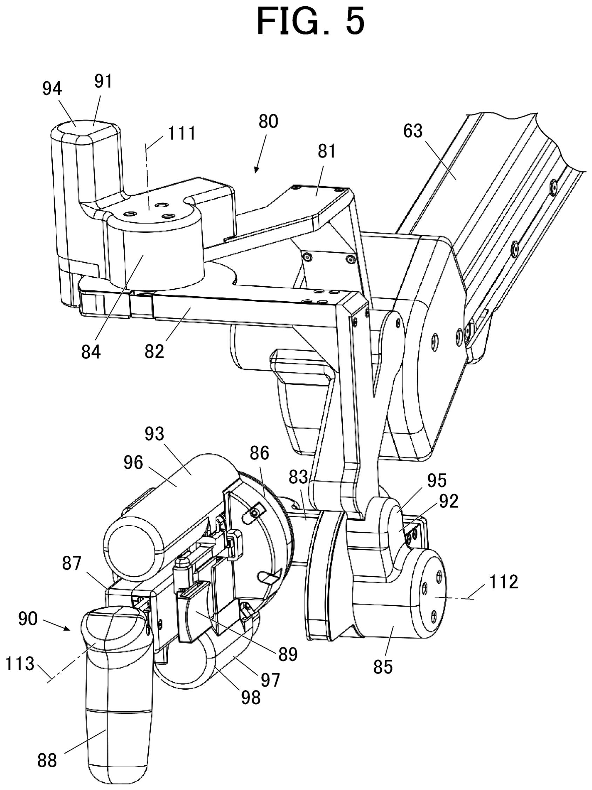

[FIG. 5] This illustrates a gimbal and a hand controller at a distal end of the right console.
[FIG. 6] This is a perspective view of the right hand controller.
[FIG. 7] This is a perspective view of the right hand controller.
[FIG. 8] This is a side view of the right hand controller.
[FIG. 9] This is a side view of the right hand controller.

### DESCRIPTION OF EMBODIMENTS

One or more embodiments of the present disclosure are described below with reference to the drawings. The features and technical effects of the embodiments are understood from the following detailed description and drawings. However, the scope of the invention is not limited to the embodiments disclosed below. The drawings are provided for illustrative purposes only, and the scope of the invention is not limited to the examples in the drawings.

### <1. Overview of Remote-controlled Robot System>

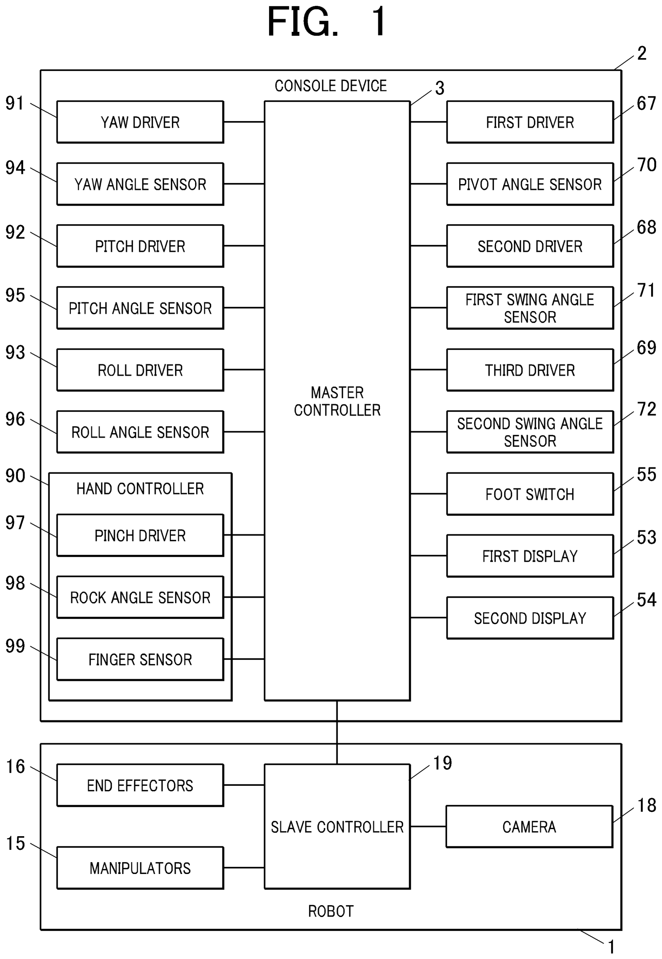

FIG. 1 is a block diagram illustrating a remote-controlled robot system.

The remote-controlled robot system includes a robot 1 and a console device 2. The console device 2 is a master and the robot 1 is a slave. When an operator, such as a doctor, manipulates the console device 2, the robot 1 operates in accordance with the operation of the console device 2.

### <2. Robot>

The robot 1 is installed in a workplace such as an operating room. The robot 1 is a robot that performs tasks such as surgery. The robot 1 includes two manipulators 15, two end effectors 16, a camera 18, a slave controller 19, and a base.

The two manipulators 15 are disposed side by side on the left and right and mounted to the base. Each of the manipulators 15 is a vertical articulated robot having five degrees of freedom, six degrees of freedom, or seven degrees of freedom. The manipulator 15 includes a plurality of links, a plurality of joints, and a plurality of drivers. A proximal link among these links is coupled to the base via a pivoting joint among these joints, and these links are sequentially coupled by the joints from the proximal end to a distal end of the manipulator 15. These joints include bending and torsional joints as well as pivoting joints. The drivers are connected to the joints. The manipulator 15 is operated by the drivers applying torque to the joints. Here, the term "distal" means farther from the base, and the term "proximal" means closer to the base.

Each of the end effectors 16 is coupled to the distal end of the manipulator 15. The end effector 16 may be a medical instrument such as forceps, tweezers, scissors, or a scalpel. In the present embodiment, the end effector 16 is forceps.

The camera 18 is coupled to the base. The camera 18 may be coupled to the base via an auxiliary manipulator or link mechanism. The camera 18 may be provided in a scope. The imaging target of the camera 18 may be the manipulators 15, the end effectors 16 or a work object. The work object refers to an object to be treated by the end effectors 16. For example, the work object may be a diseased portion to be operated on.

The slave controller 19 is one or more computers having a central processing unit (CPU), a random-access memory (RAM), a bus, a bus controller, an interface circuit, a drive circuit, a communication device, and the like. The slave controller 19 receives an operation signal from a master controller 3 of the console device 2. The slave controller 19 controls the manipulators 15 and end effectors 16 according to the operation signal, thereby causing the manipulators 15 and end effectors 16 to follow consoles 60 of the console device 2.

### <3. Console device>

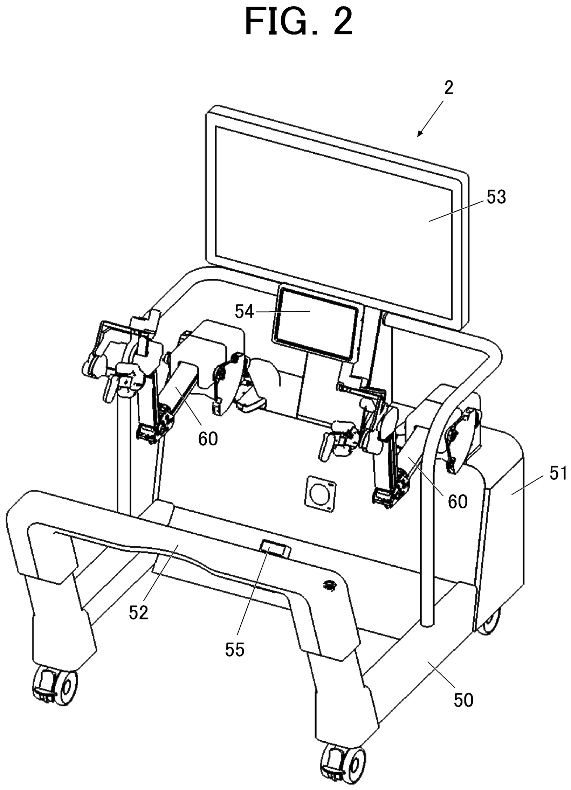

FIG. 2 is a perspective view of the console device 2.

The console device 2 is installed away from the workplace such as an operating room. The console device 2 may be installed in the workplace.

The console device 2 includes a cart 50, a base 51, a seat 52, a first display 53, a second display 54, a foot switch 55, two consoles 60, and the master controller 3.

The master controller 3 is one or more computers having a central processing unit (CPU), a random-access memory (RAM), a bus, a bus controller, an interface circuit, a drive circuit, a communication device, and the like. The master controller 3 controls the console device 2. The master controller 3 can communicate with the slave controller 19 via a network or other means.

The cart 50 includes casters with stoppers on its underside and is movable by the casters.

The base 51 is fixed to the rear of the cart 50 and stands upright from the rear of the cart 50.

The seat 52 is fixed to the front of the cart 50 and stands upright from the front of the cart 50. The operator sits on the seat 52 and manipulates the consoles 60 and the foot switch 55.

The first display 53 and second display 54 are full-color displays, such as liquid crystal displays and organic EL displays. The first display 53 and second display 54 are arranged vertically one above the other and mounted on the base 51. The display surfaces of the first display 53 and second display 54 face forward. The first display 53 displays an image captured by the camera 18. The second display 54 displays the state of the robot 1, more specifically the states of the manipulators 15, the end effectors 16, and the camera 18.

The foot switch 55 is mounted on a lower portion of base 51. When the foot switch 55 is pressed by the operator, the foot switch 55 turns on. When the foot switch 55 is released, the foot switch 55 turns off. When the foot switch 55 turns on, the foot switch 55 outputs a high-level ON signal to the master controller 3. The ON signal is a trigger. When the foot switch 55 turns off, the foot switch 55 outputs a low-level OFF signal to the master controller 3. The foot switch 55 is for turning on and off the following of the manipulators 15 and end effectors 16 relative to the consoles 60.

The two consoles 60 are coupled to an upper portion of the base 51 and arranged side by side on the left and right with a gap between them. These consoles 60 extend forward from the base 51, allowing the operator to grasp the left and right consoles 60 with the left and right hands, respectively. When the operator moves the left console 60 with the left hand, the left manipulator 15 and left end effector 16 operate following the movement of the left console 60. When the operator moves the right console 60 with the right hand, the right manipulator 15 and right end effector 16 operate following the movement of the right console 60.

### <3-1. Consoles>

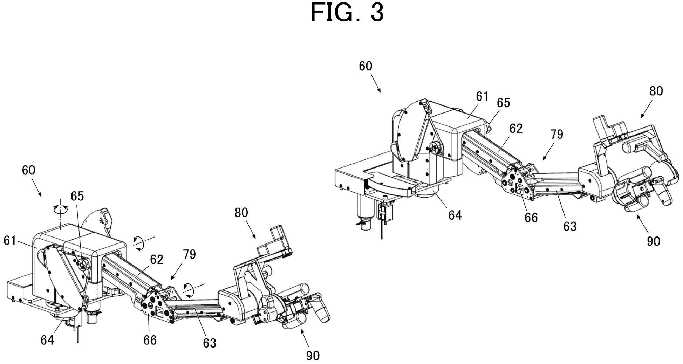

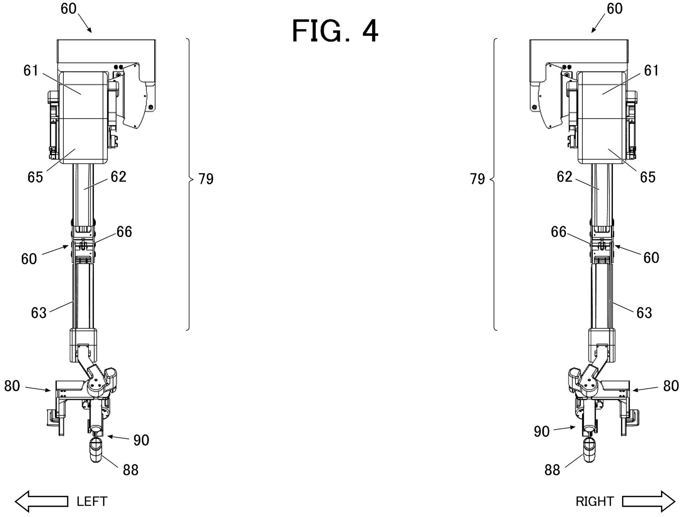

FIG. 3 is a perspective view of the left and right consoles 60. FIG. 4 is a plan view of the left and right consoles 60.

As illustrated in FIGs. 3 and 4, each of the consoles 60 includes an articulated link mechanism 79, a gimbal 80, and a hand controller 90. As illustrated in FIG. 1, the console 60 includes drivers 67 to 69 and sensors 70 to 72 that are utilized to control the articulated link mechanism 79.

A proximal end of the articulated link mechanism 79 is coupled to the base 51, a distal end of the articulated link mechanism 79 is coupled to the gimbal 80, and the hand controller 90 is coupled to the gimbal 80. Here, the term "distal" means farther from the base 51, and the term "proximal" means closer to the base 51.

The articulated link mechanism 79 supports the gimbal 80 and the hand controller 90 for translational movement with three degrees of freedom. The gimbal 80 supports the hand controller 90 for rotational movement with three degrees of freedom. When the operator grasps the hand controller 90 and moves the hand controller 90, the posture and orientation of the hand controller 90 is changed by the gimbal 80, and the hand controller 90 and gimbal 80 are translated by the articulated link mechanism 79.

The articulated link mechanism 79 includes a pivot 61, a proximal link 62, a distal link 63, a first joint 64, a second joint 65, and a third joint 66.

The pivot 61 is rotatably coupled to an upper portion of the base 51 by the first joint 64. The pivot 61 is provided so as to pivot relative to the base 51 around a vertical pivot axis via the first joint 64. The pivot 61 and the first joint 64 correspond to the proximal end of the articulated link mechanism 79.

A pivot angle sensor 70, such as a rotary encoder, is provided on the base 51, and a first driver 67, such as a motor, is provided on the base 51. The pivot angle sensor 70 and the first driver 67 are coupled to the first joint 64. The pivot angle sensor 70 detects a pivot angle of the pivot 61 around the pivot axis and outputs the detected value to the master controller 3. The master controller 3 transfers the detected value of the pivot angle sensor 70 to the slave controller 19. The slave controller 19 controls the manipulator 15 according to the detected value of the pivot angle sensor 70.

The first driver 67 applies torque around the pivot axis to the first joint 64 and the pivot 61.

A proximal end of the proximal link 62 is rotatably coupled to the pivot 61 by the second joint 65. The proximal link 62 is provided so as to swing up and down relative to the pivot 61 around a horizontal first swing axis extending left and right via the second joint 65. The proximal link 62 may be configured by a link mechanism, such as a parallel link mechanism.

A first swing angle sensor 71, such as a rotary encoder, is provided in the pivot 61, and a second driver 68, such as a motor, is provided in the pivot 61. The second driver 68 and the first swing angle sensor 71 are coupled to the second joint 65. The first swing angle sensor 71 detects a first swing angle of the proximal link 62 around the first swing axis and outputs the detected value to the master controller 3. The master controller 3 transfers the detected value of the first swing angle sensor 71 to the slave controller 19. The slave controller 19 controls the manipulator 15 based on the detected value of the first swing angle sensor 71.

The second driver 68 applies torque around the first swing axis to the second joint 65 and the proximal link 62.

A proximal end of the distal link 63 is rotatably coupled to a distal end of the proximal link 62 by the third joint 66. The distal link 63 is provided so as to swing up and down relative to the proximal link 62 around a second swing axis parallel to the first swing axis via the third joint 66. The distal link 63 may be configured by a link mechanism, such as a parallel link mechanism.

A second swing angle sensor 72, such as a rotary encoder, is provided in the pivot 61, and a third driver 69, such as a motor, is provided in the pivot 61. The second swing angle sensor 72 and the third driver 69 are coupled to the third joint 66 via a link mechanism or other means. The second swing angle sensor 72 detects a second swing angle of the distal link 63 around the second swing axis and outputs the detected value to the master controller 3. The master controller 3 transfers the detected value of the second swing angle sensor 72 to the slave controller 19. The slave controller 19 controls the manipulator 15 based on the detected value of the second swing angle sensor 72.

The third driver 69 applies torque around the second swing axis to the third joint 66 and the distal link 63.

A distal end of the distal link 63 corresponds to the distal end of the articulated link mechanism 79, and the gimbal 80 is mounted on the distal end of the distal link 63.

The gimbal 80 includes a coupling arm 81, a first rotating arm 82, a second rotating arm 83, joints 84 to 86, drivers 91 to 93, and angle sensors 94 to 96.

A proximal end of the coupling arm 81 is mounted on the distal end of distal link 63. The coupling arm 81 forms an L-shaped bend from its proximal end to its distal end within the plane in which the distal link 63 swings up and down.

A proximal end of the first rotating arm 82 is rotatably coupled to the distal end of the coupling arm 81 by the joint 84. The first rotating arm 82 is rotatable relative to the coupling arm 81 around the yaw axis 111 of the joint 84. The yaw axis 111 is along the plane in which the distal link 63 swings up and down.

A yaw angle sensor 94, such as a rotary encoder, is coupled to the joint 84, and a yaw driver 91, such as a motor, is coupled to the joint 84. The yaw angle sensor 94 detects the yaw angle of the first rotating arm 82 around the yaw axis 111 and outputs the detected value to the master controller 3. The master controller 3 transfers the detected values of the yaw angle sensor 94 to the slave controller 19. The slave controller 19 controls the manipulator 15 based on the detected value of the yaw angle sensor 94.

The yaw driver 91 applies torque around the yaw axis 111 to the joint 84 and the first rotating arm 82.

A proximal end of the second rotating arm 83 is rotatably coupled to a distal end of the first rotating arm 82 by the joint 85. The second rotating arm 83 is rotatable relative to the first rotating arm 82 around the pitch axis 112 of the joint 85. The pitch axis 112 is orthogonal to the yaw axis 111.

A pitch angle sensor 95, such as a rotary encoder, is coupled to the joint 85, and a pitch driver 92, such as a motor, is coupled to the joint 85. The pitch angle sensor 95 detects the pitch angle of the first rotating arm 82 around the pitch axis 112 and outputs the detected value to the master controller 3. The master controller 3 transfers the detected value of the pitch angle sensor 95 to the slave controller 19. The slave controller 19 controls the manipulator 15 based on the detected value of the pitch angle sensor 95.

The pitch driver 92 applies torque around the pitch axis 112 to the joint 85 and the second rotating arm 83.

The hand controller 90 is rotatably coupled to a distal end of the second rotating arm 83 by the joint 86. The hand controller 90 is rotatable relative to the second rotating arm 83 around the roll axis 113 of the joint 86. The roll axis 113, pitch axis 112, and yaw axis 111 intersect each other at a common point of intersection. A tab 87 extends from the joint 86 toward the common point of intersection.

A roll angle sensor 96, such as a rotary encoder, is coupled to the joint 86, and a roll driver 93, such as a motor, is coupled to the joint 86. The roll angle sensor 96 detects the roll angle of the tab 87 around the roll axis 113 and outputs the detected value to the master controller 3. The master controller 3 transfers the detected values of the roll angle sensor 96 to the slave controller 19. The slave controller 19 controls the manipulator 15 based on the detected value of the roll angle sensor 96.

The roll driver 93 applies torque around the roll axis 113 to the joint 86 and the tab 87.

### <3-2. Hand Controller>

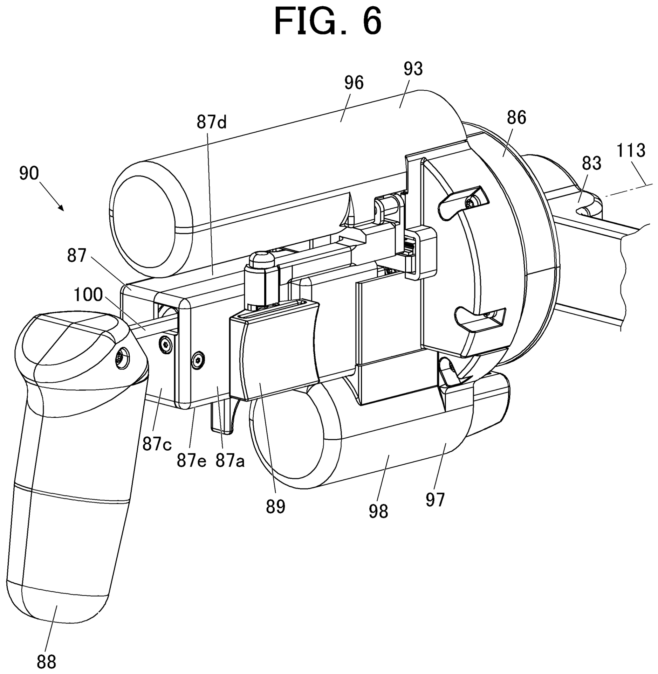

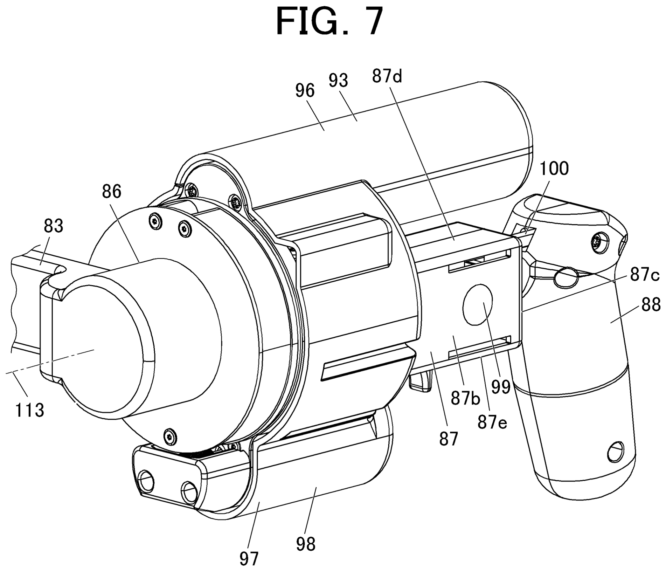

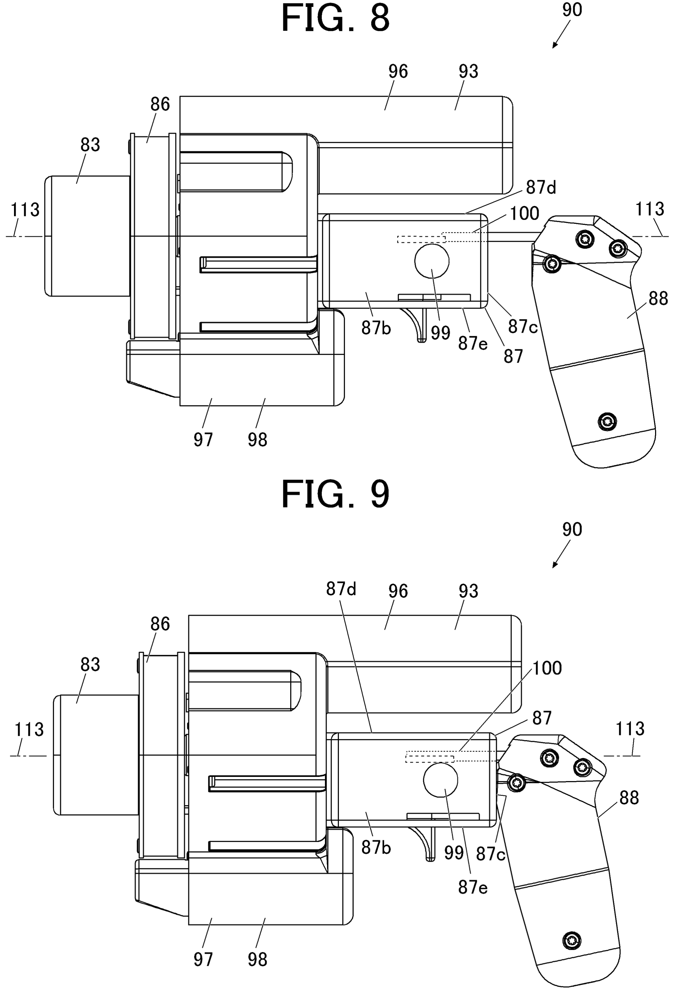

FIGs. 6 and 7 are perspective views of the hand controller 90. FIGs. 8 and 9 are side views of the hand controller 90.

The hand controller 90 includes the tab 87, a handle 88, an operation lever 89, a pinch driver 97, a rock angle sensor 98, a finger sensor 99, and a linear guide 100.

The tab 87 is rotatably coupled to the distal end of the second rotating arm 83 by the joint 86. The tab 87 is rotatable relative to the second rotating arm 83 around the roll axis 113 of the joint 86. The roll axis 113 is orthogonal to the pitch axis 112. The roll axis 113, pitch axis 112, and yaw axis 111 intersect each other at the common point of intersection. The tab 87 extends along the roll axis 113 from the joint 86 toward the common point of intersection.

The tab 87 is formed into a rectangular parallelepiped shape. The shape of the tab 87 may be cylindrical or elliptical with a central axis along the roll axis 113.

The tab 87 has a first side surface 87a, a second side surface 87b, an end surface 87c, a top surface 87d, and a bottom surface 87e. The first side surface 87a and the second side surface 87b face away from each other. The first side surface 87a and second side surface 87b extend from the joint 86 along the roll axis 113 toward the end surface 87c. They extend from the joint 86 along the roll axis 113 toward the end surface 87c. The top surface 87d is provided on the upper edge of the first side surface 87a and the upper edge of the second side surface 87b. The end surface 87c is provided between the rear edge of the first side surface 87a and the rear edge of the second side surface 87b at a distance from the joint 86 in the direction of the roll axis 113. The end surface 87c is provided between the rear edge of the top surface 87d and the rear edge of the bottom surface 87e.

The operation lever 89 is an operation element. The operation lever 89 is disposed facing the first side surface 87a of the tab 87. A proximal end of the operation lever 89 is coupled to the tab 87 so as to be rotatable around an axis orthogonal to the roll axis 113 at a point closer to the joint 86. The operation lever 89 extends along the roll axis 113 from its proximal end to its distal end. The operation lever 89 is rockable around the axis at its proximal end so as to allow the operation lever 89 to be moved in and out of contact with the first side surface 87a of the tab 87. The operator rocks the operation lever 89, primarily with the index finger, to move the operation lever 89 in and out of contact with the first side surface 87a of the tab 87.

The pinch driver 97 includes, for example, a motor. The pinch driver 97 applies torque to the operation lever 89.

The rock angle sensor 98 includes, for example, a rotary encoder. The rock angle sensor 98 detects a rock angle of the operation lever 89 and outputs the detected value to the master controller 3. The master controller 3 transfers the detected value of the rock angle sensor 98 to the slave controller 19. The slave controller 19 controls the end effector 16 based on the detected value of the rock angle sensor 98.

The finger sensor 99 is provided on the second side surface 87b of the tab 87. The finger sensor 99 is, for example, a reflective optical sensor, a capacitive touch sensor, a pressure-sensitive switch, a membrane switch, a tactile switch, a momentary switch, a dome switch, or a membrane switch. When a finger of the operator, for example, a thumb, is placed on the second side surface 87b and attached to the finger sensor 99, the finger sensor 99 turns on and detects the finger. Therefore, the finger sensor 99 outputs a high-level ON signal to the master controller 3. When the operator's finger leaves the finger sensor 99 and the second side surface 87b, the finger sensor 99 turns off and does not detect the finger. Therefore, the finger sensor 99 outputs a low-level OFF signal to the master controller 3.

The finger sensor 99 is for turning on and off the following of the manipulator 15 and end effector 16 relative to the console 60. Specifically, the finger sensor 99 is for turning off the following of the manipulator 15 and end effector 16 relative to the console 60 when the operator unexpectedly releases the hand controller 90 during the following of the manipulator 15 and end effector 16.

The linear guide 100 is mounted inside the tab 87. A portion of the linear guide 100 protrudes from the inside of the tab 87 through the end surface 87c of the tab 87 to the outside of the tab 87. The linear guide 100 guides the handle 88 along the roll axis 113.

The handle 88 is disposed facing the end surface 87c of the tab 87. The handle 88 is coupled to the linear guide 100. The handle 88 extends from the linear guide 100 in a direction intersecting the roll axis 113. The handle 88 is shaped like a column, more specifically a cylinder, having a central axis intersecting the roll axis 113. The handle 88 may be rotatable relative to the linear guide 100 around its central axis. The handle 88 may be fixed to the linear guide 100.

The handle 88 is movable along the roll axis 113 by the linear guide 100 so as to allow the handle 88 to be moved in and out of contact with the end surface 87c of the tab 87. The linear guide 100 includes a stopper that sets a movement range from the position where the handle 88 is closest to the end surface 87c of the tab 87 to the position where the handle 88 is farthest away from the end surface 87c of the tab 87.

The operator places the palm of the hand on the handle 88, grasps the handle 88 with the palm, and pinches the tab 87 and the operation lever 89 with the fingers. When the operator holds the hand controller 90 in this manner, the burden on the operator's hand during manipulation of the hand controller 90 is reduced and the operator can manipulate the hand controller 90 with precision and finesse.

The handle 88 can be moved in and out of contact with the end surface 87c of the tab 87. This enables the operator to adjust the position of the handle 88 according to the size of the hand, allowing the finger to be placed on the finger sensor 99.

The operator manipulates the pivot 61, proximal link 62, distal link 63, and gimbal 80 by moving the wrist, arm, shoulder, and upper body while grasping the handle 88. When the operator manipulates the pivot 61, proximal link 62, distal link 63, and gimbal 80, the master controller 3 transfers the detected values of the pivot angle sensor 70, first swing angle sensor 71, second swing angle sensor 72, yaw angle sensor 94, pitch angle sensor 95 and roll angle sensor 96 to the slave controller 19. The slave controller 19 controls the manipulator 15 based on the detected values of the pivot angle sensor 70, first swing angle sensor 71, second swing angle sensor 72, yaw angle sensor 94, pitch angle sensor 95 and roll angle sensor 96. This allows the left manipulator 15 to follow the operation of the left console 60 and the right manipulator 15 to follow the operation of the right console 60.

When the operator rocks the operation lever 89 by finger, the master controller 3 transfers the detected value of the rock angle sensor 98 to the slave controller 19. The slave controller 19 controls the end effector 16 based on the detected value of the rock angle sensor 98. This allows the left end effector 16 to follow the rocking of the operation lever 89 of the left hand controller 90 and the right end effector 16 to follow the operation of the operation lever 89 of the right hand controller 90.

### <4. Modes>

The master controller 3 and the slave controller 19 switch between an operation mode, a standby mode, and a safe mode.

### <4-1. Operation Mode>

When the master controller 3 and slave controller 19 are in the operation mode, the master controller 3 can control the manipulator 15 and end effector 16 through the slave controller 19. Specifically, the master controller 3 transfers the detected values of the pivot angle sensor 70, first swing angle sensor 71, second swing angle sensor 72, yaw angle sensor 94, pitch angle sensor 95 and roll angle sensor 96 to the slave controller 19. The slave controller 19 controls the manipulator 15 based on the detected values of the pivot angle sensor 70, first swing angle sensor 71, second swing angle sensor 72, yaw angle sensor 94, pitch angle sensor 95 and roll angle sensor 96 without ignoring the signals from the master controller 3. This allows the left manipulator 15 to follow the operation of the left console 60 and the right manipulator 15 to follow the operation of the right console 60.

The master controller 3 transfers the detected value of the rock angle sensor 98 to the slave controller 19. The slave controller 19 controls the end effector 16 based on the detection of the rock angle sensor 98 without ignoring the signal from the master controller 3. This allows the left end effector 16 to follow the rocking of the operation lever 89 of the left hand controller 90 and the right end effector 16 to follow the operation of the operation lever 89 of the right hand controller 90.

### <4-2. Standby Mode>

When the master controller 3 and slave controller 19 are in the standby mode, the master controller 3 can control the manipulators 15 and end effectors 16 through the slave controller 19. Specifically, even when the master controller 3 transfers the detected values of the pivot angle sensor 70, first swing angle sensor 71, second swing angle sensor 72, yaw angle sensor 94, pitch angle sensor 95 and roll angle sensor 96 to the slave controller 19, the slave controller 19 ignores the signals from the master controller 3. Therefore, even when the operator manipulates the left and right consoles 60, the left and right manipulators 15 will not operate.

Even when the master controller 3 transfers the detected value of the rock angle sensor 98 to the slave controller 19, the slave controller 19 ignores the signal from the master controller 3. Therefore, even when the operator rocks the operation levers 89 of the left and right hand controllers 90, the left and right end effectors 16 will not operate.

By the master controller 3 controlling the first driver 67 based on the detected value of the pivot angle sensor 70, the first driver 67 generates resisting torque on the first joint 64 and the pivot 61 that is balanced by the torque applied by the operator to the pivot 61. For example, the master controller 3 performs feedback control of the first driver 67 based on the detected value of the pivot angle sensor 70 such that the change in the pivot angle of the pivot 61 in the first joint 64 becomes zero. Therefore, even when the operator tries to pivot the left and right consoles 60, the left and right consoles 60 are fixed and will not pivot.

Similarly, by the master controller 3 controlling the second driver 68, the second driver 68 generates resisting torque on the second joint 65 and the proximal link 62 that is balanced by the torque applied by the operator to the proximal link 62. By the master controller 3 controlling the third driver 69, the third driver 69 generates resisting torque on the third joint 66 and the distal link 63 that is balanced by the torque applied by the operator to the distal link 63. By the master controller 3 controlling the yaw driver 91 based on the yaw angle sensor 94, the yaw driver 91 generates resisting torque on the joint 84 and the first rotating arm 82 that is balanced by the torque applied by the operator to the first rotating arm 82. By the master controller 3 controlling the pitch driver 93 based on the pitch angle sensor 95, the yaw driver 91 generates resisting torque on the joint 85 and the second rotating arm 83 that is balanced by the torque applied by the operator to the second rotating arm 83. By the master controller 3 controlling the roll driver 93 based on the roll angle sensor 96, the roll driver 93 generates resisting torque on the joint 86 and the tab 87 of the hand controller 90 that is balanced by the torque applied by the operator to the tab 87. Therefore, even when the operator tries to move the left and right consoles 60 and the left and right gimbals 80, the left and right consoles 60 and the left and right gimbals 80 are fixed and will not move.

By the master controller 3 controlling the pinch driver 97 based on the detected value of the rock angle sensor 98, the pinch driver 97 generates resisting torque on the operation lever 89 that is balanced by the torque applied by the operator to the operation lever 89. For example, the master controller 3 performs feedback control of the pinch driver 97 based on the detected value of the rock angle sensor 98 such that the change in the rock angle of the operation lever 89 becomes zero. Therefore, even when the operator tries to turn the operation levers 89 of the left and right hand controllers 90, the operation levers 89 are fixed and will not turn.

### <4-3. Safe Mode>

When the master controller 3 and slave controller 19 are in the safe mode, the master controller 3 can control the manipulators 15 and end effectors 16 through the slave controller 19. The processing of the master controller 3 in the safe mode is the same as that of the master controller 3 in the standby mode. The processing of the slave controller 19 in the safe mode is the same as that of the slave controller 19 in the standby mode.

### <5. Mode Switching>

### <5-1. In Standby Mode>

Initially, the master controller 3 and the slave controller 19 are in the standby mode.

The master controller 3 calculates the logical product of the signals of the left and right finger sensors 99 and the signal of the foot switch 55. The master controller 3 determines whether to switch from the standby mode to the operation mode based on the logical product.

When the operator places his/her fingers on the left and right finger sensors 99, the master controller 3 receives ON signals from the left and right finger sensors 99. In this case, when the operator presses the foot switch 55 with his/her foot and the master controller 3 receives an ON signal from the foot switch 55, the logical product of the signals of the left and right finger sensors 99 and the signal of the foot switch 55 is "true". Therefore, the master controller 3 switches from the standby mode to the operation mode. When the master controller 3 switches to the operation mode, the master controller 3 transmits information indicating the operation mode to the slave controller 19. This causes the slave controller 19 to switch from the standby mode to the operation mode.

On the other hand, when the operator releases his/her finger from either or each of the left and right finger sensors 99, the master controller 3 receives an OFF signal from either or each of the left and right finger sensors 99. In this case, even when the operator presses the foot switch 55 with his/her foot and the master controller 3 receives an ON signal from the foot switch 55, the logical product of the signals of the left and right finger sensors 99 and the signal of the foot switch 55 is "false". Therefore, the master controller 3 maintains the standby mode. The slave controller 19 also maintains the standby mode.

Therefore, when the operator properly holds the hand controllers 90, the operator can manipulate the manipulators 15 and end effectors 16 using the consoles 60 by pressing the foot switch 55. In a case where the operator does not properly hold the hand controllers 90, the operator can manipulate neither the manipulators 15 nor the end effectors 16 using the consoles 60 even when the operator presses the foot switch 55.

### <5-2. In Operation Mode>

When the master controller 3 and slave controller 19 switch to the operation mode, the operator has his/her fingers on the left and right finger sensors 99. Therefore, the master controller 3 receives ON signals from the left and right finger sensors 99.

The master controller 3 calculates the negative logical product of the signals of the left and right finger sensors 99. The master controller 3 determines whether to switch from the operation mode to the safe mode based on the negative logic product.

When the operator keeps his/her fingers on the left and right finger sensors 99, the master controller 3 receives ON signals from the left and right finger sensors 99. The negative logical product of the signals of the left and right finger sensors 99 is "false". Therefore, the master controller 3 maintains the operation mode. The slave controller 19 also maintains the operation mode.

When the operator releases his/her finger from either or each of the left and right finger sensors 99, the master controller 3 receives an OFF signal from either or each of the left and right finger sensors 99. The negative logical product of the signals of the left and right finger sensors 99 becomes "true". Therefore, the master controller 3 switches from the operation mode to the safe mode. When the master controller 3 switches to the safe mode, the master controller 3 transmits information indicating the safe mode to the slave controller 19. This causes the slave controller 19 to switch from the operation mode to the safe mode. Therefore, even when the operator accidentally releases the hand controller 90, the manipulator 15 and end effector 16 are fixed and do not operate.

Furthermore, the master controller 3 calculates the logical product of the signals of the left and right finger sensors 99 and the signal of the foot switch 55. The master controller 3 determines whether to switch from the operation mode to the standby mode based on the logical product.

When the operator keeps his/her fingers on the left and right finger sensors 99, the master controller 3 receives ON signals from the left and right finger sensors 99. In this case, when the operator presses the foot switch 55 with his/her foot and the master controller 3 receives an ON signal from the foot switch 55, the logical product of the signals of the left and right finger sensors 99 and the signal of the foot switch 55 is "true". Therefore, the master controller 3 switches from the operation mode to the standby mode. When the master controller 3 switches to the standby mode, the master controller 3 transmits information indicating the standby mode to the slave controller 19. This causes the slave controller 19 to switch from the operation mode to the standby mode. On the other hand, when the operator does not press the foot switch 55 with his/her foot while keeping his/her fingers on the left and right finger sensors 99, the logical product of the signals of left and right finger sensors 99 and the signal of the foot switch 55 is "false". Therefore, the master controller 3 maintains the operation mode.

### <5-3. In Safe Mode>

When the master controller 3 and slave controller 19 switch to the safe mode, the operator releases his/her finger from either or each of the left and right finger sensors 99. Therefore, the master controller 3 receives an OFF signal from either or each of the left and right finger sensors 99.

When the master controller 3 and the slave controller 19 switch to the safe mode, the master controller 3 starts timing. During timing, the master controller 3 calculates the logical product of the signals of the left and right finger sensors 99. During timing, the master controller 3 determines whether to switch from the safe mode to the operation mode based on the logical product.

During timing, when the operator places his/her fingers on the left and right finger sensors 99, the master controller 3 receives ON signals from the left and right finger sensors 99. The logical product of the signals of the left and right finger sensors 99 is "true". Therefore, the master controller 3 switches from the safe mode to the operation mode. When the master controller 3 switches to the operation mode, the master controller 3 stops timing and resets the elapsed time. The master controller 3 then transmits the information indicating the operation mode to the slave controller 19. This causes the slave controller 19 to switch from the safe mode to the operation mode. Therefore, when the operator holds the hand controllers 90 properly again, the operator can manipulate the manipulators 15 and end effectors 16 using the consoles 60.

On the other hand, when the operator keeps his/her fingers released from either or each of the left and right finger sensors 99 during timing, the master controller 3 receives an OFF signal from either or each of the left and right finger sensors 99. The logical product of the signals of the left and right finger sensors 99 is "false". Therefore, the master controller 3 maintains the safe mode and continues timing.

During timing, the master controller 3 determines whether to switch from the safe mode to the standby mode based on the signal from the foot switch 55. Therefore, when the operator presses the foot switch 55 with his/her foot and the master controller 3 receives an ON signal from the foot switch 55, the master controller 3 switches from the safe mode to the standby mode. When the master controller 3 switches to the standby mode, the master controller 3 stops timing and resets the elapsed time. The master controller 3 then transmits the information indicating the standby mode to the slave controller 19. This causes the slave controller 19 to switch from the safe mode to the standby mode.

While the operator keeps his/her finger released from either or each of the left and right finger sensors 99 without pressing the foot switch 55, the time measured by the master controller 3 reaches a predetermined time such as 5 seconds. Then, the master controller 3 switches from the safe mode to the standby mode. When the master controller 3 switches to the standby mode, the master controller 3 stops timing and resets the elapsed time. The master controller 3 then transmits the information indicating the standby mode to the slave controller 19. This causes the slave controller 19 to switch from the safe mode to the standby mode. Therefore, the operator can manipulate neither the manipulators 15 nor the end effectors 16 unless the operator properly holds the hand controllers 90 and presses the foot switch 55 again.

The predetermined time is not limited to 5 seconds and may be less than 5 seconds or may be 5 seconds or more.

### <6. Advantageous effects>

(1) The operator places the palm of the hand on the handle 88, grasps the handle 88 with the palm, and pinches the tab 87 and the operation lever 89 with the fingers. This reduces the burden on the operator's hand during manipulation of the hand controller 90. This also allows the operator to manipulate the hand controller 90 with precision and finesse. The operator can move the operation lever 89 with his/her finger while holding the hand controller 90.
(2) The handle 88 can be moved in and out of contact with the end surface 87c of the tab 87. This enables the operator to grasp the handle 88 with his/her palm and place his/her finger on the finger sensor 99, regardless of the size of the operator's hand. The finger of the operator's hand is detected by the finger sensor 99, regardless of whether the operator's hand is large or small.
(3) When the master controller 3 and slave controller 19 are in the standby mode, in a case where the operator does not properly hold the hand controller 90, the operator's finger is not detected by the finger sensor 99. In such a case, the master controller 3 and slave controller 19 do not switch to the operation mode even when the operator presses the foot switch 55. Therefore, the operator cannot manipulate the manipulators 15 and end effectors 16 using the consoles 60. On the other hand, in a case where the operator properly holds the hand controller 90, the operator's finger is detected by the finger sensor 99. In such a case, the operator can manipulate manipulators 15 and end effectors 16 using the consoles 60 by pressing the foot switch 55.
(4) When the master controller 3 and slave controller 19 are in the operation mode, in a case where the operator accidentally releases the hand controller 90, the finger is not detected by the finger sensor 99. The master controller 3 and slave controller 19 switch to the safe mode, and the consoles 60, manipulators 15, and end effectors 16 are fixed and will not move. This contributes to preventing erroneous manipulation of the consoles 60, manipulators 15, and end effectors 16.
(5) When the master controller 3 and the slave controller 19 are in the operation mode, in a case where the operator accidentally releases the hand controller 90 and then immediately holds the hand controller 90 properly again, the finger is detected by the finger sensor 99. In such a case, the master controller 3 and slave controller 19 return from the safe mode to the operation mode. This allows the operator to continue to manipulate the consoles 60, manipulators 15 and end effectors 16.
(6) When the master controller 3 and slave controller 19 are in the operation mode, in a case where the operator accidentally releases the hand controller 90 and leaves the hand controller 90 released, the master controller 3 and slave controller 19 switch to the standby mode. This contributes to preventing erroneous manipulation of the consoles 60, manipulators 15, and end effectors 16.

### Reference Signs List

- 2: console device
- 3: master controller
- 55: foot switch
- 70: pivot angle sensor
- 71: first swing angle sensor
- 72: second swing angle sensor
- 79: articulated link mechanism
- 87: tab
- 87a: first side surface
- 87b: second side surface
- 87c: end surface
- 88: handle
- 89: operation lever
- 90: hand controller

## Claims

1. A hand controller comprising:
a tab that has:
a first side surface and a second side surface facing away from the first side surface; and
an end surface that extends from the first side surface to the second side surface;
a handle that faces the end surface of the tab and is mounted to the tab to allow the handle to be moved in and out of contact with the end surface of the tab;
an operation element that faces the first side surface of the tab and is coupled to the tab to allow the operation element to be moved in and out of contact with the first side surface of the tab; and
a finger sensor that detects a finger of a hand of an operator, the finger sensor being on the second side surface of the tab.

2. The hand controller according to claim 1, wherein:
the handle is grasped by a palm of the hand; and
the tab and the operation element are pinched by the finger of the hand.

3. The hand controller according to claim 1, wherein:
when the finger of the hand is placed on the second side surface, the finger sensor detects the finger of the hand; and
when the finger of the hand is released from the second side surface, the finger sensor does not detect the finger of the hand.

4. A console device comprising:
the hand controller according to claim 1;
a foot switch that outputs a trigger when pressed by a foot of the operator;
an articulated link mechanism that has:
a pivotable proximal end; and
a distal end to which the hand controller is connected,
the articulated link mechanism translatably supporting the hand controller; and
a controller that switches between an operation mode in which the controller is capable of controlling a robot and a standby mode in which the controller is incapable of controlling the robot, wherein
when the finger sensor detects the finger of the hand and the controller receives the trigger from the foot switch, the controller switches the robot between the operation mode and the standby mode.

5. The console device according to claim 4, wherein, when the controller is in the standby mode and the controller receives the trigger from the foot switch while the finger sensor is detecting the finger of the hand, the controller switches from the standby mode to the operation mode.

6. The console device according to claim 4, wherein, when the controller is in the standby mode and the controller receives the trigger from the foot switch while the finger sensor is not detecting the finger of the hand, the controller maintains the standby mode.

7. The console device according to claim 4, wherein, when the controller is in the operation mode and the controller receives the trigger from the foot switch while the finger sensor is detecting the finger of the hand, the controller switches from the operation mode to the standby mode.

8. The console device according to claim 4, wherein, when the controller is in the operation mode and the finger sensor no longer detects the finger of the hand, the controller switches from the operation mode to a safe mode in which the controller is incapable of controlling the robot.

9. The console device according to claim 8, wherein, when the finger sensor detects the finger of the hand before a predetermined time elapses from a time at which the finger sensor starts not to detect the finger of the hand, the controller switches from the safe mode to the operation mode.

10. The console device according to claim 8, wherein, when the finger sensor has not detected the finger of the hand for the predetermined time since a time at which the finger sensor starts not to detect the finger of the hand, the controller switches from the safe mode to the standby mode.

11. The console device according to claim 8, 9, or 10, further comprising a driver that applies a torque to a joint of the articulated link mechanism, wherein, when the controller is in the safe mode, the controller controls the driver to apply a resisting torque to the joint of the articulated link mechanism.

12. The console device according to claim 11, wherein the controller controls the driver such that the resisting torque is balanced by a torque generated at the joint of the articulated link mechanism by a manipulation of the operator.

13. The console device according to any one of claims 4 to 10, further comprising a driver that applies a torque to a joint of the articulated link mechanism, wherein, when the controller is in the standby mode, the controller controls the driver to apply a resisting torque to the joint of the articulated link mechanism.

14. The console device according to claim 13, wherein the controller controls the driver such that the resisting torque is balanced by a torque generated at the joint of the articulated link mechanism by a manipulation of the operator.
